(19) Europäisches Patentamt
European Patent Office
Office européen des brevets

(11) Publication number: 0 316 117 A1

# (12) EUROPEAN PATENT APPLICATION

(21) Application number: 88310391.3

(22) Date of filing: 04.11.88

(51) Int. Cl.4: C07F 9/10 , C07F 9/09 , C07F 9/58 , A61K 31/66

(30) Priority: 06.11.87 IL 84387

(43) Date of publication of application: 17.05.89 Bulletin 89/20

(84) Designated Contracting States: AT BE CH DE ES FR GB GR IT LI LU NL SE

(71) Applicant: SCIENSCOPE INTERNATIONAL N.V.
6 John B. Gorsiraweg P.O. Box 3889
Curaçao(AN)

(72) Inventor: Shenfeld, Avner
16 Hesse Street
Rehovot(IL)

(74) Representative: Mercer, Christopher Paul
Carpmaels & Ransford 43, Bloomsbury Square
London WC1A 2RA(GB)

## (54) Antiviral compounds, compositions containing such compounds and methods of treatment.

(57) An invention relating to novel compounds and compositions of matter for use in alleviating the symptoms of a number of diseases, especially those caused by viral infection of mammals, including humans, is disclosed. Compositions of the present invention contain as an active ingredient novel derivatives of the phospholipid type which are characterized in that a chemical group not found in naturally occurring phospholipids is covalently bound to the phosphate group of the phosphatidyl residue. The invention also discloses compounds and pharmaceutical compositions for therapeutic use and a therapeutic method for treating a number of diseases, especially those related to viral infections.

EP 0 316 117 A1

# ANTIVIRAL COMPOUNDS, COMPOSITIONS CONTAINING SUCH COMPOUNDS AND METHODS OF TREATMENT

## FIELD OF INVENTION

The invention relates to novel compounds and compositions of matter, for use in alleviating the symptoms of a number of diseases, especially those caused by viral infection of mammals, including humans. Compositions of the present invention contain as an active ingredient novel derivatives of the phospholipid type which are characterized in that a chemical group not found in naturally occurring phospholipids is covalently bound to the phosphate group of the phosphatidyl residue. This invention also relates to the compounds and pharmaceutical compositions for therapeutic use and to a therapeutic method for treating a number of diseases, especially those related to viral infections.

The repeated administration of such compositions alleviates in an appreciable manner the symptoms of various viral afflictions and produces a pronounced positive effect on the well-being of patients.

## BACKGROUND OF INVENTION

Viral infections in mammals, and especially in humans, are very widespread and in spite of the considerable progress in chemotherapy, little progress has been made in creating specific drugs which either cure viral diseases or alleviate symptoms of patients afflicted with these. Certain diseases, for example, Acquired Immune Deficiency Syndrome (AIDS), Herpes, Cytomegalovirus (CMV) infection,and Influenza, among others, in which encapsulated or envelope viruses are responsible for the disease state, remain without any effective cure or even any means to produce an appreciable improvement of the state of the patient. The present invention relates to novel compounds and compositions for reducing the infectivity of encapsulated viruses.

Encapsulated viruses contain membranes possessing characteristics of ordinary biological membranes. The membranes of these viruses contain a substantial phospholipid component. As such, these membranes are amenable to extraction or incorporation of lipids, in and out of the membrane.

Most previous attempts to reduce viral infectivity through the envelope lipids were carried out by increasing lipid fluidity. The main approach was the extraction of membrane cholesterol. Vesicular Stomatitis Virus (VSV) was found to lose infectivity upon extraction of membrane cholesterol in a reversible manner. It has been proposed that in the cholesterol depleted membrane the increased fluidity reduces the protrusion of the VSV antigen. The human immunodeficiency virus (HIV) extraction of membrane cholesterol with AL721 (a mixture of egg lipids) has been shown to reduce the extent of infection of a leukemia line derived from T4 lymphocytes. These observations prompted the application of AL721 for the treatment of AIDS patients and sporadic claims have been made for the reduction in HIV titer in patients who received such treatment.

Fluidization of viral membranes by incorporation of the lipid soluble preservative butylated hydroxytoluene (BHT) has similarly been found to inactivate various envelope viruses. Similar results have been found by membrane fluidization with lipid solvents, for example, with phospholipase or by detergents. Direct inhibitory effects of lipids on viruses have also been observed with the stearic acid derivative CONTRACAN™, and certain ether phospholipid analogues of lecithin and lysolecithin. Synthetic acyl-substituted glycero phosphate esters have previously not been reported or proposed as anti-viral agents.

It is an object of the present invention to provide novel compositions comprising the reaction product of a natural acyl substituted glycerophosphate and an alcohol to produce a synthetic acyl-substituted glycerophosphate ester. These compounds are effective anti-viral agents.

It is a further object of this invention to provide novel synthetic methods for producing the compounds of the present invention.

It is an additional object of this invention to provide therapeutic compositions comprising an effective amount of the synthetic compounds of the present invention.

It is also an object of this invention to provide a novel therapeutic method for treating humans infected with encapsulated viruses, especially the HIV virus.

It is a further object of this invention to provide a method of prophylactically treating individuals at high risk of developing AIDS.

## BRIEF DESCRIPTION OF THE INVENTION

The compounds of the present invention are useful as anti-viral agents to reduce the infectivity of viruses, especially encapsulated viruses such as HIV and Herpes, and consequently alleviate certain symptoms of the viral disease state. Compounds of the present invention comprise the reaction product of a natural acyl-substituted glycerophosphate and an alcohol or thiol to produce a synthetic acyl-substituted glycerophosphate ester. These compounds are effective in reducing the activity of encapsulated viruses. The synthetic compounds of the present invention are designed to be incorporated into the phospholipid membrane of the virus and to produce dynamic and structural changes in the membrane. These changes in the viral membrane are expected to register for a long period of time and may be responsible for the reduced activity and virulence of viruses exposed to the synthetic compounds.

The compounds of the invention are of use in combatting viral infections which afflict humans and offer great potential against afflictions which remain without any means of alleviation of symptoms or potential cure. The compounds of the present invention are based on phospholipids having substituent groups that are not found in naturally occurring phospholipids.

Compounds of the present invention are based on the following general formula:

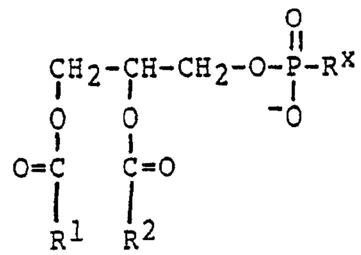

wherein $R^1$ and $R^2$ are aliphatic chains of natural fatty acids and $R^x$ is an alcohol or thiol radical of the type which produces a composition effective to reduce the infectivity of encapsulated viruses. Preferably, Rx is an alcohol radical.

The compounds of the present invention are designed to be incorporated into the phospholipid compartment of the viral capsule. It is believed that this results in a change in the structure and dynamics of the viral membrane lipid layer which may in turn affect the expression and activity of viral surface antigens and the modulation of viral activity.

Encapsulated viruses, unlike mammalian cells, generally lack lipase enzymes. It is believed that this lack of lipase enzyme activity provides the necessary selectivity in utilizing the phospholipids of the present invention for anti-viral therapy. Host cells infected with the virus are capable of minimizing the effects of the synthetic phospholipids of the present invention due to the existence of phospholipases. These phospholipases and other lipases of the host cell can efficiently degrade incorporated synthetic phospholipids and rectify any membrane aberration that they impose on the host cell. Since viruses lack these lipases, they cannot destroy the synthetic phospholipids. Thus, the synthetic phospholipids of the present invention may exert a lasting deleterious effect on the viral capsule and produce a selective reduction in the viability and activity of the encapsulated viruses.

Based on this rationale, a number of synthetic phospholipids are presented which provide unique anti-viral activity. The synthetic compounds of the present invention are active against encapsulated viruses having a substantial phospholipid component, for example, HIV, Herpes, Sendai, Cytomegalovirus (CMV), Influenza, Parainfluenza, Epstein-Barr Virus (EBV) and Vesicular Stomatitis Virus (VSV), among others. The present invention also relates to methods for producing the compounds of the present invention. In one method for producing the compounds of the present invention an enzymatic approach to the synthesis is provided. A second method relates to the chemical synthesis of certain of these compositions.

The present invention also relates to therapeutic compositions comprising an effective amount of one or more of the compounds of the present invention optionally in the presence of an excipient or in admixture with a natural phospholipid or phosphatidic acid. Preferred therapeutic compositions include the crude reaction product produced using an enzymatic synthesis.

The present invention also relates to therapeutic methods for treating viral infections comprising administering effective amounts of the therapeutic compositions to prophylactically treat humans at high risk of developing viral infections.

DETAILED DESCRIPTION OF THE INVENTION

The present invention relates to compounds of the general formula:

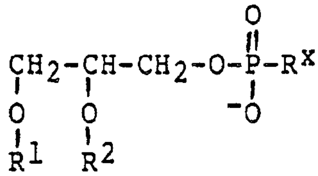

wherein $R^1$ and $R^2$ are independently hydrogen or an acyl residue of a natural fatty acid and $R^x$ is an alcohol or thiol residue of the type which produces a compound effective to reduce the activity of encapsulated viruses. Alcohol or thiol residues which are useful in the compounds of the present invention may be selected from chemically disparate groups and may include residues having pyridinium groups, phenyl or benzyl groups, including analogues of salicylic acid, acetyl salicylic acid and acetaminophen, and Schiff bases of menadione, oxidised catechol derivatives and ubiquinone, among others.

In general, the most preferred compounds of the present invention include those in which $R^x$ is derived from a naturally occurring compound but when esterified to the natural acyl-substituted glycerophosphate produces a synthetic non-natural phospholipid. Compounds of this type are designed to be less toxic and to be used as prophylactic anti-viral agents. In these preferred compounds, should the $R^x$ group be cleaved as a result of the action of the host cell's phospholipases, a biologically tolerated by-product will be produced. Thus, the use of these compounds will minimize toxicity problems and may, advantageously, provide a beneficial by-product such as a vitamin or therapeutically active compound.

The present invention preferably includes compounds wherein $R^x$ is of the general structure $-G-(A)_z-R^3$. Preferred compounds of the present invention are therefore represented by the general formula I

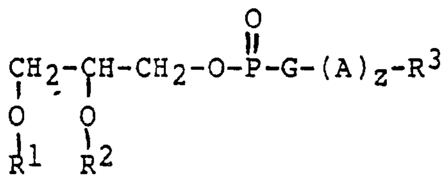

where $R^1$ and $R^2$ are independently H or an acyl residue of a natural fatty acid provided that both $R^1$ and $R^2$ are not H;
A may be $-CH_2-$, a chain of methylene groups, or a chain of methylene groups in which one or more of the methylene groups within the chain has been replaced by -O- or -S- and wherein any one of the methylene groups is optionally substituted with one or two alkyl, alkoxy or alkylthio groups;
z may be an integer such that the total number of carbon, oxygen and sulphur atoms in the linear chain is form 0 to 18;

$R^3$ may be 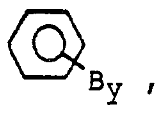 , 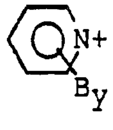 , 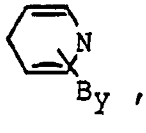 ,

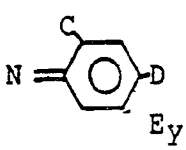 , 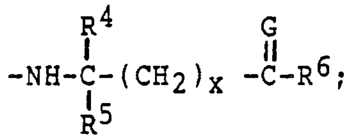 ;

or $-NH-CO-R^7$
wherein:

y is an integer from 0 to 3;
each B is independently halo, alkyl, hydroxyalkyl, aminoalkyl, $-CH(NH_2)-CH_2OH$, $-CH(NHCH_3)-CH_2OH$, hydroxyl, carboxyl, carboxamido, carboxyalkylamido, carboxydialkylamido, amino, alkyamino, dialkylamino, carboxamino, cyano or nitro;
C or D is =O and the other group is hydrogen or one of the groups E;
either each E is independently a group Y or one group P is a group Y and the other two groups form a fused benzene ring at the 5, 6 position on the ring;
G is O or S;
$R^4$, $R^5$ and $R^6$ are independently hydrogen or alkyl; and $R^7$ is an alkyl group.

Preferably both $R^1$ and $R^2$ are acyl groups.

The group $(A)_z$ may be $-CH_2-$, $-CH_2CH_2-$ or $-CH_2CH_2CH_2-$.

When $R^3$ is

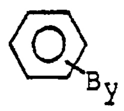

it may be derived from the known active compounds benzyl alcohol, salicylic acid, acetyl salicylic acid or acetaminophen.

When the group $R^3$ is

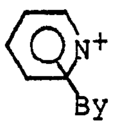

or

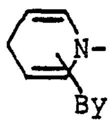

it may be attached to the group $(A)_z$ via the nitrogen atom or via a ring carbon atom. Where the hydogenated pyridinium group is attached to the phosphate group via a ring carbon atom, the nitrogen atom may be alkylated. Compounds containing the pyridinium group may be reduced in the host by endogeneous nicotinamide reductases, depending on the structure of the pyridinium group and may exist in equilibrium withthe reduced form. It is preferable to ensure that the nitrogen atom has a positive charge, either by alkylating the nitrogen atom or by ensuring that it is protonated.

The compounds of the present invention wherein the group $R^3$ is

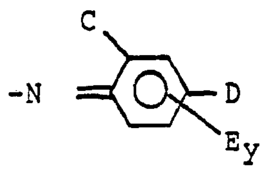

may be derived by reacting a natural phospholipid, phosphatidyl ethanolamine, with a quinone to form a Schiff's Base. Preferably the quinone is derived from a natural substance such as catechol, catecholamine, L-dopa, epinephrine, norepinephrine or menadione, among others.

The present invention also provides a method for producing the compounds of the present invention.

The process may comprise reacting a compound of the general formula II

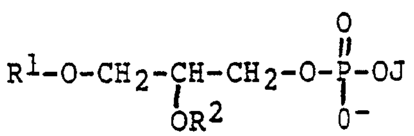

(II)

with a compound of the general formula III
$JO-(A)_z-R^3$ (III)
in the presence of an esterification catalyst, wherein $R^1$, $R^2$, $R^3$, A and z are as defined above and J is

hydrogen or a leaving group.

The esterification catalyst may be a coupling agent which is designed to produce an activated phosphate species which can phosphorylate the compound of general formula III, such as dicyclohexylcarbodiimide or a derivative thereof or a phospholipase. The phospholipase may be preferably derived from cabbage leaves. Two general methods, one chemical, one enzymatic, are therefore employed.

In both the chemical and enzymatic methods, in order to drive the reaction to completion and to reduce the competition from water, it is preferred that the compound of the general formula III is present at a high concentration and in a large molar excess as compared to the compound of the general formula II.

In any event, it is likely that there will be produced a proportion of phosphatidic acid. This may be separated from the desired product, or may be incorporated as a carrier in the pharmaceutical compositions of the present invention.

The crude reaction product of the enzymatic reaction contains natural phospholipid, synthetic phospholipid and phosphatidic acid. The advantage of this mixture is that it may be utilized in the crude form, the natural phospholipid and phosphatidic acid serving as excipients in the crude mixture. Alternatively, the compound of the present invention may be separated by purification on silica gel.

Compounds of the invention in which the group $R^3$ is

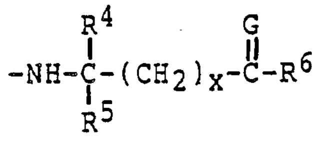

may be produced by reacting a compound of the general formula IV

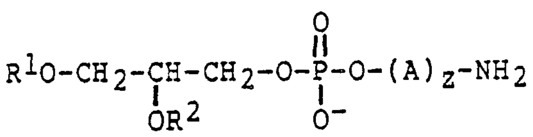

(IV)

with a compound of the general formula V

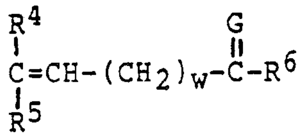

(V)

wherein $R^1$ to $R^6$, A, z, and G are as defined above and w is an integer from 0 to 5, preferably 1.

Compounds of the present invention in which $R^3$ is

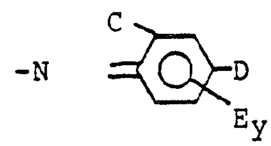

may be produced by reacting a compound of the general formula IV as defined above with a compound of the general formula VI

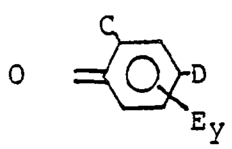

(VI)

wherein C, D, E and y are as defined above.

Throughout this description and the claims, the term "alkyl" when used alone or as part of another

radical denotes a straight chain or branched chain alkyl radical having from 1 to 6 carbon atoms.

The present invention also includes pharmaceutical compositions for use in treating patients with viral infections or at high risk for contracting a viral infection comprising an effective amount of a compound according to the present invention as the active ingredient. Preferably, the active ingredient is formulated in admixture with a pharmaceutically acceptable carrier. Preferably the pharmaceutical composition is in orally-administrable form. Compositions to be administered parenterally or in suppository form are also contemplated by the present invention.

To prepare the pharmaceutical compositions of the present invention, one or more of the compounds of the invention may preferably be intimately admixed with a pharmaceutically acceptable carrier according to conventional pharmaceutical compounding techniques, which carrier may take a wide variety of forms depending on the form of preparation desired for administration, e.g. oral or parenteral, such as intramuscular or intravenous. In preparing the compositions in oral dosage form, any of the usual pharmaceutical media may be used. Thus, for liquid oral preparations such as suspensions, elixirs and solutions, suitable carriers and additives including water, glycols, oils, alcohols, flavouring agents, preservatives, colouring agents and the like. For solid oral preparations such as powders, capsules, tablets and for solid preparations such as suppositories, suitable carriers and additives including starches, sugars, diluents, granulating agents, lubricants, binders, disintegrating agents and the like. If desired the tablets or capsules may be sugar- or enteric-coated by standard techniques. The compositions of the present invention may also be administered topically, especially for treating Herpes. Topical formulations may include a salve or cream base.

For parenterals, the carrier will usually comprise sterile water or aqueous sodium chloride solution, though other ingredients such as for the purpose of aiding dispersion may be included. Of course, where sterile water is to be used and maintained as sterile, the compositions and carriers must also be sterilized. Injectable suspensions may also be prepared, in which case appropriate liquid carriers, suspending agents and the like may be employed.

The pharmaceutical compositions of a treatment dose of the present invention may generally contain from about 10 to 500mg of the active ingredient. Preferably, about 100 to 500 mg of the active ingredient is administered. Different concentrations and amounts may, of course, be used, depending on the route of administration.

Preferably, the compositions further comprise a natural phospholipid or phosphatidyl acid as a diluent, which may be present as side products of the chemical or enzymatic method of synthesis. The ratio of the active ingredient to natural phospholipid or phosphatidic acid may vary widely, depending on the ability of the phospholipase enzyme to accomodate the substrates. Generally, this ratio ranges between about 1:99 to about 9:1.

Advantageously, the present compositions will further comprise an immunostimulant. Suitable immunostimulants include thymulin, thymosin $\alpha_1$, thymosin $\beta_4$, thymopoietin and thymopentin. The use of an immunostimulant is of particular advantage where the disease to be treated has a depressive effect on the patient's immune system. A composition including an immunostimulant will be able to combat the infection by attacking the virus and stimulating the patient's immune system so that secondary infections do not overcome the patient while he is recovering from the viral infection. These compositions are therefore particularly useful in treating patients infected with HIV.

The compounds and compositions of the present invention may be used to treat viral infections of mammals and in particular of humans. They will be of particular use in the treatment of infections caused by envelope viruses. Such envelope viruses include HIV, Sendai, CMV, Herpes, EBV and VSV. The compounds will generally be administered in doses of 100 to 500mg up to three times a day. Preferably the compounds will be administered orally but they may also be administered parenterally, topically or in suppository form. Topical formulations are especially preferred for treating Herpes infections.

The compounds of the invention may also be used prophylactically to prevent infection or to prevent the occurence of clinical symptoms associated with the viral infection. Thus, the present invention also encompasses methods for the therapeutic or prophylactic treatment of viral infections caused by envelope viruses which comprise administering to a patient in need of such treatment an amount of a compound according to the invention effective to alleviate or abolish the viral infection.

In the prophylactic treatment of the present invention, it is preferred that the $R^x$ phosphate substituent should be a compound which itself is non-toxic to the host. It is therefore preferable that compositions to be used for prophylactic treatment contain an $R^x$ phosphate substituent which is a vitamin or vitamin derivative, or a derivative of a biologically tolerated by-product or a natural substrate of biological pathways in the host.

The present invention is now described, purely by way of illustration, in the following examples. It will be understood by the skilled person that these examples are in no way limiting and that variations of detail

can be made without departing from the spirit and scope of the present invention.

I. Esterification of the Phosphate Group in Mono- and Di-Acyl Glycerophosphates

In general, the compositions of the present invention may be synthesized by esterification of the phosphate group by two different methods. In both methods, the alcohol ($R^x$) which is to be esterified on the phosphate group of the natural phospholipid is first synthesized. Once the esterification alcohol is synthesized, it is esterified onto the phosphate enzymatically or chemically.

In the enzymatic method, the alcohol is esterified in an aqueous buffered solution containing phospholipase D enzyme isolated from Savoy cabbage. Enzymatic esterification proceeds until the reaction is complete. Generally, the method of Eibel and Kovatchev, Methods in Enzymology, 72, 632 (1981) is followed. The reaction proceeds in an aqueous buffer solution containing 0.1M NaOAc and $CaCl_2$ at a pH of 5.6. Generally, the alcohol is placed in the presence of enzyme and an equivolume mixture of a phospholipid mixture diethyl ether is added (10-100 mg of phospholipid per ml of diethyl ether).The phospholipids may be purchased or obtained by the extraction of lipids from hen egg yolks by the method of Bligh and Dyer, Canadian Journal of Biology and Physiology, 37, 911 (1959), followed by the evaporation of organic solvents and precipitation of phospholipid with acetone. The phospholipid is then mixed with diethyl ether and added to the enzyme system. The reaction mixture is refluxed at a temperature of 37-40 °C. The reaction may be followed by thin layer chromatography (chloroform-methanol-water, 65-25-4 v/v) on fluorescent silica gel. Reaction products vary between about 15% and 40% based on the weight of the acyl glycerophosphate. The reaction mixture is then taken to dryness and may be used as the crude mixture or may alternatively be purified by preparative thin layer chromatography.

In the chemical method, the product is formed by esterification of the acyl glycerophosphate with the alcohol in the presence of a coupling agent, preferably DCC. The acyl glycerophosphate (phosphatidic acid analogue) may be purchased from Sigma Chemical Corporation (St. Louis, Missouri) or other biochemical supplier or alternatively, may be obtained by dephosphorylation of a phospholipid mixture (obtained as above, by extraction of phospholipids from egg yolks) in phospholipase.

In general, the acyl glycerophosphate is activated in the presence of a slight molar excess of DCC in dimethylformamide (DMF). After activation (usually after about ten minutes), esterification is then effected by the addition of a slight excess of the alcohol. The reaction is followed by fluorescent TLC on silica gel or by filtering off and weighing the dicyclohexyl urea that is formed during the reaction. After the reaction is completed, water is added and the mixture is then evaporated to dryness under reduced pressure. The mixture is then lypholized (2 x 100 ml) to remove excess solvents.

The esterification alcohol may be synthesized by following general chemical reaction principles which are readily known to those of skill in the art. The following examples which provide the synthesis of certain esterification alcohols are representative of these routine methods.

Other acyl glycerophosphate esters may be chemically synthesized by derivatizing natural phospholipids, for example phosphatidylethanolamine, in which the $R^x$ group contains a reactive moiety such as an amine or a hydroxyl group which may be further derivatized to a final product.

The synthesis of certain acyl glycerophosphate esters based on the above-described general chemical and enzymatic schemes are presented below.

Example 1

Phosphatidyl-2-hydroxyethyl 1-nicotinamide (P-HEN)

Nicotinamide was dissolved in excess 2-chloroethanol. The reaction was heated and allowed to reflux for 8 hours. The hot solution was poured into heated 95% ethanol and the product 2-hydroxyethyl 1-nicotinamide chloride (HEN) crystallized from solution upon cooling. For the enzymatic transphorphatidylation reaction pure HEN may be necessary to produce a reasonable yield. HEN may be purified by recrystallization from 95% ethanol.

The enzymatic transphosphatidylation of HEN to the phosphatidyl analogue P-HEN proceeds using the general method of Eibel and Kovatchev disclosed above. The reaction is heated under reflux and the product, P-HEN, is isolated by TLC on fluorescent silica gel (chloroform-methanol-water 65-25-4 v/v, Rf of about 0.4). The reaction yields a mixture of products containing about 20% P-HEN. Pure P-HEN may be

prepared by preparative TLC. Alternatively, P-HEN may be prepared by esterifying acyl-substituted glycerophosphate with HEN in the presence of DCC by the above-described general chemical method.

Example 2

Phosphatidyl benzyl alcohol (P-BA)

This compound was synthesized by the transphosphatidylation of lecithin in the presence of benzyl alcohol as per the synthesis of P-HEN using the above-described enzymatic method. Alternatively, the general DCC coupling method may also be used.

Example 3

4-N-Acetyl phosphatidylethanolamine (A-PE)

Phosphatidylethanolamine is dissolved in excess acetic anhydride and stirred in the absence of moisture for up to two hours. Water is added to the reaction mixture and the reaction is allowed to stir for a further hour. The mixture is then evaporated several times (95% ethanol) and lyophilized (2x) to remove any excess acetic acid.

Example 4

N-(1,1-dimethyl 3-oxybutyl phosphatidyl ethanolamine (DMOB-PE)

Phosphatidylethanolamine (PE) (Makor Chemicals Ltd. Jerusalem, Israel) was dissolved in excess mesityloxide sealed under nitrogen and incubated (bath temperature 40° C) for one week in the dark. The production of DMOB-PE was followed by TLC ($CHCl_3$-methanol-$H_2O$ 65-25-4 v/v) to confirm the reaction.

Example 5

5-Schiff Base of PE and Menadione (Vitamin $K_3$) (PE-M)

An equimolar amount of PE and menadione (10 mM of each) were mixed in chloroform. Schiff base product was indicated by the appearance of a reddish color. The reaction was complete after one hour. Excess solvent was evaporated to produce PE-M.

Example 6

A pharmaceutical composition containing the compound P-HEN was produced by dispersing the sterilized mixture of P-HEN and other natural phospholipids and phosphatidic acids obtained by the enzymatic process in sterile water. The P-HEN/phospholipid mixture was evaporated to dryness, mixed with the sterile water and homogenized by Polytrone ™ (Kinematic GMbH, Switzerland) for five minutes to disperse the mixture. The dispersion thus formed was diluted with sterile water to produce an orally acceptable composition containing 100 mg of P-HEN in each 5 ml aliquot.

Examples 7 - 11

Pharmaceutical compositions containing P-BA, A-PE, DMOB-PE and PE-M are produced by combining

an effective amount of the synthetic phospholipid in admixture with an excipient and formulated into tablet, powder or suppository form. Alternatively, a sterile suspension may be produced by following the procedure for Example 6 above.

II. Anti-Viral Testing

Example 12

Effectiveness of DMOB-PE, PE-M, P-HEN and P-BA on Parainfluenza Virus

The effectiveness of certain novel compounds was tested against Parainfluenza virus which was grown in fertilized hen eggs as determined by the method of Haywood, J. of Mol. Biol., 83, 427 (1974). The virus was at titer of $10^5$ to $10^6$ was incubated in the presence of 10 ug/ml or 100 ug/ml of one of the four compounds at 37° C for 2 hours. The mixtures of the virus and anti-viral compound were diluted to a final concentration of $10^{-7}$ then injected into 9-day old fertilized hen eggs using 5 to 6 eggs for each dilution and incubated at 37° C for 48 hours. After the incubation period, a drop of amniotic fluid from the eggs was put on mirror-glass and mixed with 2% washed hen erythrocytes. Appearance of hemagglutination indicated the presence of virus in the egg. $LD_{50}$ was calculated from the results. The following Table 1 presents the results that were obtained. The virus concentrations ranged from -(undetectable) to + + + + (indicative of hemagglutination produced by virus grown under control conditions).

Table 1

| | Virus Concentrations | | | |
|---|---|---|---|---|
| Compound | $10^4$ | $10^3$ | $10^2$ | 10 |
| DMOB-PE 10 µg/ml | ++++ | ++ | + | ± |
| DMOB-PE 100 µg/ml | ++ | + | - | - |
| PE-M 10 µg/ml | +++ | + | - | - |
| PE-M 100 µg/ml | + | - | - | - |
| P-HEN 10 µg/ml | ++ | ± | - | - |
| P-HEN 100 µg/ml | - | - | - | - |
| P-BA 100 µg/ml | + | - | - | - |
| Control (virus only) | ++++ | ++++ | ++++ | ++++ |

Example 13

P-HEN was further assayed on vesicular stomatitis virus (VSV), Sendai virus, and the Human Immunodeficiency Virus (HIV). VSV was assayed according to the method of Moore et al., (J.Virol. 27 320, 1978). Sendai virus was assayed according to the method described for Parainfluenza in Example 7. HIV was assayed according to the following method. Serial dilutions of P-HEN were pre-incubated for 2 hours at 37° C with 1:10 dilution of a stock of HIV-1 obtained from CEM-HIV supernatant. MT4 cells (a human T4 leukemia cell line) were added to a final concentration of $3x10^5$ cells per ml. Each 3 or 4 days, the cell suspensions were counted and diluted 3 to 4 fold to adjust the cell concentration back to the original level. The HIV titer was assessed at days 6 to 10 by scoring the appearance of syncitia followed by cell death.

The results of this set of tests are summarized in Table 2.

Table 2

Effect of P-HEN on Several Envelope Viruses

| P-HEN Conc. | HIV Level | Sendai Level[b] | VSV Level |
|---|---|---|---|
| 20 µg/ml | 0[a] | 2 | 3 |
| 50 µg/ml | 0 | 3 | 4 |
| 200 µg/ml | 0 | 2 to 0 | 4 to 0 |
| 500 µg/ml | 0 | 2 to 3 | 5 to 0 |

a no virus detected after 10 days in culture.

b the number of orders of magnitude the virus level was reduced.

Example 14

P-HEN, DMOB-PE, and A-PE were further assayed at various concentrations on Sendai virus, as described in Example 7. The results of the assay appear in Table 3 below. Activity appears as a percentage which reflects the reduction in the virus titer compared to control.

Table 3

| Compound | Conc. | Virus Titer Reduction |
| --- | --- | --- |
| P-HEN | 1-200µg/ml | Range of activity between 70% to nò virus detected. Only one sample (10ug/ml) below 90% reduction of virus titer. |
| DMOB-PE | 100µg/ml | 100% (virus undetectable) |
| | 50µg/ml | ranged from 90-93% |
| | 10µg/ml | ranged from 75-99% |
| | 1µg/ml | less than 10% |
| A-PE | 100µg/ml | 100% (virus undetectable) |
| | 50µg/ml | ranged from 90-99% |
| | 10µg/ml | ranged from 75-95% |
| | 1µg/ml | about 50% |

Example 15

In Vivo Study of the Anti-Viral Effect of P-HEN on HIV

P-HEN was evaluated in vivo to determine its anti-viral effect on HIV. The assay used in the study was the infectivity of the MT4 cell line (T4 Type LeukemiaCell Line) as measured by the formation of syncitia or, in certain cases, reverse transcriptase activity. P-HEN was delivered to the cells as emulsions. Emulsions were prepared by lyophilizing or evaporating solutions of P-HEN to dryness and then adding RPMI solution without protein to a final concentration of 5 or 10 mg/ml in a glass tube. The mixture produced. an emulsion after sonication for 5 minutes. P-HEN was delivered to the infected cells in varying concentrations by serially diluting the sonicated standard emulsions.

HIV-1 virus at a titer of $10^5$ to $10^6$ infective units (ten-fold dilution of HIV-1 virus obtained from a EM-HIV-1 supernatant was preincubated for 2 hours at 37°C in the presence of P-HEN at concentrations of 50, 100, 200 and 500 ug/ml. After incubation, MT4 cells were added at final concentration of $3x10^5$ cells/ml. Each 3 or 4 days, the cell suspensions were counted and diluted 3 or 4 times to adjust the cell concentration. Syncitia appeared in the cell culture after days 5 to 7 in MT4 cells infected with HIV.

Three general types of tests were run to determine the anti-viral activity of P-HEN to HIV-1 and the relative toxicity of P-HEN to the host cells. In tests 1 and 2, the anti-viral and toxic effect of a single dose of P-HEN were determined. In tests 1 and 2, a portion of the cells were washed to remove the emulsion of P-HEN and HIV-1. In those cells not washed, P-HEN and HIV from the original emulsion were diluted as a consequence of normal cell dilution. In test 1, the separation and washing of cells occurred on day 4 and, in test 2, the separation and washing occurred on day 2.

In test 3, the effect of adding the P-HEN at different times was determined. In test 3, P-HEN was added to the cells in a pre-incubation step (1 or 2 hours before infection) or at various times after infection (see

Table 6).

Viability and infectivity of HIV were determined by syncitia formation and reverse transcriptase activity. In brief, reverse transcriptase activity was assayed using a synthetic template primer and a tritiated radiolabelled substrate in vivo. The amount of virus was determined by the amount of radiolabelled precipitated material at the end of the reaction. Reverse transcriptase assay was used to determine, in the absence of syncitia, that MT4 cells were producing HIV.

Tables 4-6, appended to the specification, represent the results of the three different tests carried out. Syncitia formation is presented as + or -symbols and range from - (no syncitia formation) to + + maximum syncitia formation). The performance of the reverse transcriptase assay is indicated by RT. The results of the reverse transcriptase assay are represented as RT+ or RT- depending on the presence of reverse transcriptase activity. Absence of cell viability is presented as a T or as a Tox depending upon whether the absence of cell viability is understood to be the natural expiration of cell viability or the toxicity of the P-HEN.

In sum, the tests performed showed that P-HEN exhibited significant anti-HIV activity and relatively low toxicity in the MT4 leukemia cell line.

## Example 16

### Clinical Study of Anti-Viral Effects of P-HEN on AIDS Patients

A clinical study was conducted on 15 patients who evidenced measurable clinical and laboratory signs associated with HIV infection. Ten of the patients were either asymptomatic antigen positive or exhibited minor symptoms of AIDS. The remaining 5 patients were severe cases of AIDS, all 5 having undergone Azidothymidine (AZT) therapy without success. These latter five cases were considered desperate cases. Accordingly, most patients demonstrated clinical signs compatible with mild to moderate AIDS-Related Complexes (ARC) and serum HIV antigenemia together with reduced $T_4$ lymphocytes measured according to $CD_4$+ antigen. Some patients, however, did not show significant antigenemia, while other patients were borderline AIDS cases.

All 15 patients were placed on the following escalated dosage schedule:

1st week- 100 mg/day/patient
2nd week- 100 mg/day (total 200 mg)
3rd week and on- 100 mg + 200mg/day (total 300 mg).

P-HEN was packaged in vials containing 100 mg active material in 5 ml aqueous solution and administered orally, avoiding concomitant ingestion of fats.

Patients were monitored by clinical observation and laboratory tests. The main hallmarks of deterioration in the clinical status of the AIDS patient in the order of occurrence and importance are: fatigue, weight loss, diarrhea, fevers, generalized lymphadenopathy and oral candidiasis. The presence of opportunistic infections such as Kaposi's sarcoma, lymphoma or signs of acute encephalopathy indicate that the patient has entered the stage of overt AIDS. A chart was devised in which all of the above signs and symptoms were monitored.

The following laboratory tests were conducted to monitor progression of the disease state:

a) HIV antigenemia in serum by reversed ELISA (Abbott).
b) White blood cell counts with differentials.
c) T cell populations, using fluorescent monoclonal antibodies.
d) Routine hematology (including platelets) and routine biochemical evaluation.

Of the 15 patients treated, 14 demonstrated a clear-cut improvement in status as evidenced by improvements in their mental, physical and social states. Of the 5 patients who failed to respond to AZT therapy, 4 of the patients showed a clear cut decrease in blood levels of HIV, 3 patients showed an increase in T4 cell count and one patient showed no evidence of detectable HIV antigen in the blood.

It is to be understood by those skilled in the art that the foregoing description and examples are illustrative of practicing the present invention, but are in no way limiting. Variations of the detail presented herein may be made without departing from the spirit and scope of the present invention.

Viral Infectivity and Cell Viability

Table 4

Test 1 - Emulsion Wash at Day 4

| Day | 7 | 8 | 9 | 10 | 11 | 12 | 13 | 15 | 18 | 21 | 24 | 28 |
|---|---|---|---|---|---|---|---|---|---|---|---|---|
| MT4 + HIV-1 | ++ | ++ | ++ | ++ | ++RT+ | ++ | ++ | ++ | ++RT+ | T | T | T |
| MT4 + HIV-1 + P-HEN | | | | | | | | | | | | |
| 1000ug/ml | T | T | ? | ? | +?RT- | +? | - | (+)RT- | -RT- | (+)RT- | (+) | T+- |
| 500ug/ml | T | -RT- | - | + | +RT- | (+) | + | ++RT- | ++ | T | T | T |
| 250ug/ml | - | -RT- | + | +(+) | +RT- | + | ++ | ++ | ++ | T | T | T |

Test 1 - Progressive Cell Dilution

| Day | 7 | 8 | 9 | 10 | 11 | 12 | 13 | 15 | 18 | 21 | 24 | 28 |
|---|---|---|---|---|---|---|---|---|---|---|---|---|
| MT4 + HIV-1 | ++ | ++RT(+) | ++ | ++ | ++RT+ | ++ | ++ | ++RT+ | ++ | ++RT+ | T | T |
| MT4 + HIV-1 + P-HEN | | | | | | | | | | | | |
| 1000ug/ml | T | T | T | T | -RT- | ? | - | (+)?RT- | -RT- | +RT(+) | (-) | (+ |
| 500ug/ml | T | -RT- | - | - | -RT- | ? | (+) | -?RT- | ++RT- | ++RT+ | ++ | T |
| 250ug/ml | - | -RT- | - | - | -RT- | (+) | (+) | +RT- | ++ | ++ | ++ | T |

Viral Infectivity and Cell Viability

Table 5 Cont'd

Test 2 - Progressive Cell Dilution

| Day | 5 | 7(8) | 9 | 10 | 12 | 14 | 16 | 19 | 22 | 26 | 28 | 30 | 33 |
|---|---|---|---|---|---|---|---|---|---|---|---|---|---|
| MT4 + HIV-1 | - | + | ++ | ++ | ++ | T | T | T | T | T | T | T | T |
| MT4 + HIV-1 + P-HEN | | | | | | | | | | | | | |
| 500ug/ml | TCX | - | - | - | - | - | - | - | - | - | - | - | - |
| 200ug/ml | - | - | - | - | - | - | - | - | - | - | - | - | - |
| 100ug/ml | - | - | - | - | - | - | - | +/- | + | T/+ | T | T | T |
| 50ug/ml | - | - | - | - | - | - | - | - | -/-? | - | - | - | - |

## Viral Infectivity and Cell Viability

Table 5

Test 2 - Emulsion Wash at Day 2

| Day | 5 | 7(8) | 9 | 10 | 12 | 14 | 16 | 19 | 22 | 26 | 28 | 30 | 33 |
|---|---|---|---|---|---|---|---|---|---|---|---|---|---|
| MT4 + HIV-1 | - | + | ++ | ++ | ++ | T | T | T | T | T | T | T | T |
| MT4 + HIV-1 + P-HEN | | | | | | | | | | | | | |
| 500ug/ml | TOX | - | - | - | - | - | - | (+) | - | - | - | - | - |
| 200ug/ml | - | - | - | - | - | - | (+) | +/- | - | - | - | - | - |
| 100ug/ml | - | - | - | - | - | - | (+) | + | T/++ | T | T | T | T |
| 50ug/ml | - | - | - | - | +/-? | - | (+) | +/- | - | - | - | - | - |

Viral Infectivity and Cell Viability

Table 6

Test 3 - Emulsion added at Different Times

| PREINCUBATION 2H IH | INFECTION (DAY) 0 3 7 | CULTURE TIME(DAY) | 6 | 7 | 8 | 9 | 10 | 13 | 15 | 17 | 20 |
|---|---|---|---|---|---|---|---|---|---|---|---|
| ├──┤ | | | – | – | – | – | – | – | – | – | – |
| ├──┤ | ├─┤ (0–3) | | – | – | – | – | – | – | – | – | – |
| ├──┤ | ├───┤ (0–7) | | – | – | – | ++/– | ++ | ++ | T | T | T |
| | ├─┤ (0–3) | | – | – | – | +/– | +/1 | + | + | T | T |
| | ├───┤ (0–7) | | – | – | – | – | – | – | – | – | – |
| Control | HIV with wash | | (+) | (+) | (+) | +/– | ++/– | T/– | T/– | T/– | T/– |
| Control | HIV without wash | | (+) | (+) | + | ++ | T | T | T | T | T |

## Claims

1. A compound for use as an anti-viral agent comprising the reaction product of a natural acyl substituted glycerophosphate and an alcohol or thiol to produce a synthetic acyl-substituted glycerophosphate ester effective to reduce the activity of envelope viruses.

2. A compound according to claim 1 of the general formula I

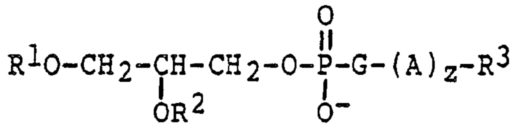

(I)

wherein:

$R^1$ and $R^2$ are independently hydrogen or an acyl residue derived from a natural fatty acid, provided that $R^1$ and $R^2$ are not both hydrogen;

A is $-CH_2-$, a chain of methylene groups, or a chain of methylene groups in which one or more of the methylene groups within the chain has been replaced by -O- or -S-and wherein any one of the methylene groups is optionally substituted with one or two alkyl, alkoxy or alkylthio groups;

z is an integer such that the total number of carbon, oxygen and sulphur atoms in the linear chain is from 0 to 18;

$R^3$ is 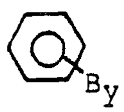 , 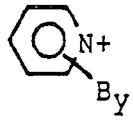 , 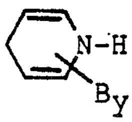 ,

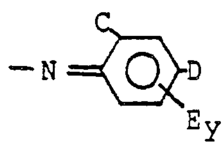 , 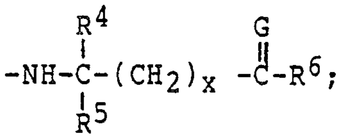 ; or

3. A compound according to claim 2, wherein both $R^1$ and $R^2$ are acyl groups.

4. A compound according to claim 2 or claim 3, wherein G is 0.

5. A compound according to any one of claims 2 to 4, wherein the group $(A)_z$ is $-CH_2-$, $-CH_2CH_2-$ or $-CH_2CH_2CH_2-$or one of these groups wherein one or more of the methylene groups is substituted with one alkyl group.

6. A compound according to claim 5, wherein the group $(A)_z$ is $-CH_2-$.

7. A compound according to claim 5, wherein group $(A)_z$ is $-CH_2CH_2-$.

8. A compound according to any one of claims 2 to 7, wherein $R^3$ is

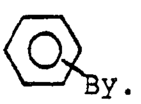

9. A compound according to claim 8, wherein $y = 0$.

10. A compound according to claim 8, wherein $y = 2$, one group B is hydroxy, the other group B is carboxyalkyl and the groups B are ortho to one another.

11. A compound according to claim 8, wherein $y = 2$, one group B is hydroxy, the other group B is alkylcarboxylamino and the groups B are para to one another.

12. A compound according to any one of claims 2 to 7 wherein the group $R^3$ is

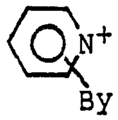 or 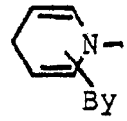.

and the group $R^3$ is attached to the group $(A)_z$ via the nitrogen atom.

13. A compound according to any one of claims 2 to 7, wherein the group $R^3$ is

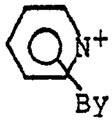 or 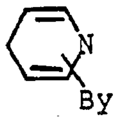

the group $R^3$ is attached to the group $(A)_z$ via a ring carbon atom, and the nitrogen atom is substituted with an alkyl group.

14. A compound according to claim 12, wherein the group $R^3$ is

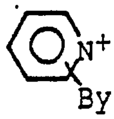

wherein y is 1 and the group B is carboxamide, carboxalkylamido or carboxdialkylamido.

15. A compound according to any one of claims 2 to 7, wherein the group $R^3$ is

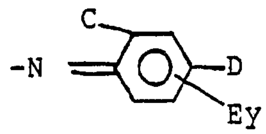.

16. A compound according to claim 15, wherein the group D is =O.

17. A compound according to claim 15 or claim 16, wherein y=3 two of the groups E form a fused benzene ring on the 5,6 position of the ring and the third group E is an alkyl group.

18. A compound according to any one of claims 2 to 7 wherein the group $R^3$ comprises

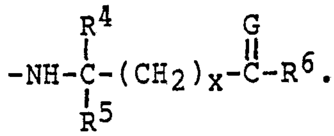.

19. A compound according to claim 18, wherein G is 0 and x is 1.

20. A compound according to any one of claims 2 to 7, wherein $R^3$ is $-NH-CO-R^7$ and $R^7$ is an alkyl group.

21. A compound according to claim 4, wherein the groups $-(A)_z-R^3$ together comprise:

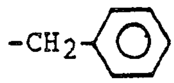 ; 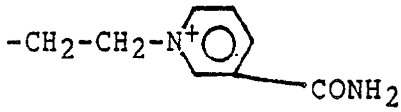 ,

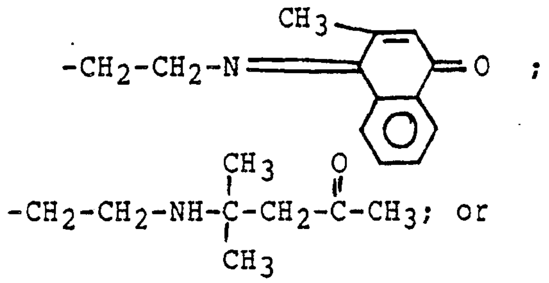 ; or

$-CH_2-CH_2-NH-CO-CH_3$.

22. A method for producing a compound according to any one of claims 2 to 21 which comprises reacting a compound of the general formula II

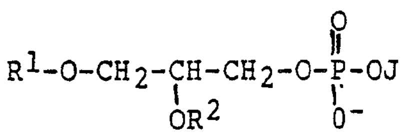 (II)

with a compound of the general formula III

$JO-(A)_z-R^3$ (III)

in the presence of an esterification catalyst, wherein $R^1$, $R^2$, $R^3$, A and z are as defined in claim 2 and J is hydrogen or a leaving group.

23. The method of claim 22, wherein the esterification catalyst is dicyclohexycarbodiimide or a derivative thereof.

24. The method of claim 22, wherein the esterification catalyst is a phospholipase.

25. The method of claim 24, wherein the phospholipase is derived from cabbage leaves.

26. The method of any one of claims 22 to 25, wherein the method of general formula III is present in a large molar excess as compared to the compound of the general formula II.

27. A method for producing a compound according to claim 18, which comprises reacting a compound of the general formula IV

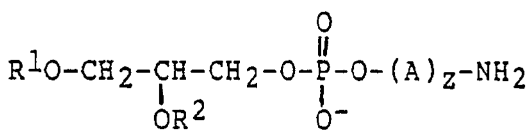 (IV)

with a compound of the general formula V

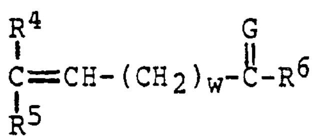 (V)

wherein $R^1$ to $R^6$, A, z, and G are as defined in claim 2 and w is an integer from 0 to 5.

28. A method for producing a compound according to claim 15, which comprises reacting a compound of the general formula IV as defined in claim 27 with a compound of the general formula VI

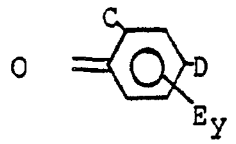

(VI)

wherein C, D, E and y are as defined in claim 2.

29. A pharmaceutical composition comprising a compound according to any one of claims 1 to 21 as the active ingredient and admixture with a pharmaceutically acceptable carrier.

30. The composition of claim 29, wherein the pharmaceutical composition is in orally-administrable form.

31. The composition of claim 29 or claim 30, which further comprises a natural phospholipid or phosphatidyl acid.

32. The composition of claim 31, wherein the ratio of the active ingredient to natural phospholipid or phosphatidic acid is about 1:4.

33. The composition of any one of claims 29 to 32, which further comprises an immunostimulant.

34. The composition of any one of claims 29 to 33, wherein the group $(A)_z$-$R^3$ is derived from a vitamin.

35. A compound according to any one of claims 1 to 21 or a composition according to any one of claims 29 to 33 for use in treating viral infections caused by envelope virus.

36. A compound according to any one of claims 1 to 21 or a composition according to any one of claims 29 to 34 for use in treating HIV infections.

37. The composition of claim 34 for use in the prophylactic treatment of patients at risk of being infected by envelope viruses such as HIV.

38. A method of preparing a composition according to any one of claims 29 to 34, which comprises mixing a compound according to any one of claims 1 to 21 with a pharmaceutically acceptable carrier and, optionally, with a natural phospholipid, phosphatidyl acid or immunostimulant.

European Patent Office

# PARTIAL EUROPEAN SEARCH REPORT

which under Rule 45 of the European Patent Convention shall be considered, for the purposes of subsequent proceedings, as the European search report

Application number: EP 88 31 0391

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 169 812 (CIBA-GEIGY AG)<br>* Pages 8,9,58-68 * | 1-5,7, 20-38 | C 07 F 9/10<br>C 07 F 9/09<br>C 07 F 9/58<br>A 61 K 31/66 |
| X | EP-A-0 009 088 (F. HOFFMAN-LA ROCHE & CO.)<br>* Pages 3,8,9 * | 1-6,8, 9,21 | |
| Y | EP-A-0 174 912 (CIBA-GEIGY AG)<br>* Pages 48-52 * | 1,29-38 | |

TECHNICAL FIELDS SEARCHED (Int. Cl.4)

C 07 F 9/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 2-38

Claims searched incompletely: 1

Claims not searched:

Reason for the limitation of the search:

The formulation of claim 1 is too general, making a complete search impossible.
In claim 2, there is no given definition of G, R4,R5, R6, R7 or By.
The search has been done according to the definition given in page 8 of the description.

| Place of search | Date of completion of the search | Examiner |
|---|---|---|
| The Hague | 19-01-1989 | BESLIER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82